# FASCICULE DE BREVET EUROPEEN

(11) **EP 0 676 386 B1**
(45) Date de publication et mention de la délivrance du brevet: **09.06.1999**
(21) Numéro de dépôt: 95200801.9
(22) Date de dépôt: 30.03.1995
(51) Int. Cl.: C07C 17/38, C07C 17/00, C07C 19/08

(54) **Procédé de séparation du 1,1-difluoroéthane de ses mélanges avec le fluorure d'hydrogène**
Verfahren zur Abtrennung von 1,1-Difluorethan aus seinen Mischungen mit Fluorwasserstoff
Process for de separation of 1,1-difluoroethane from its mixtures with hydrogen fluoride

(30) Priorité: 11.04.1994 FR 9404354
(43) Date de publication de la demande: 11.10.1995
(73) Titulaire: SOLVAY (Société Anonyme), B-1050 Bruxelles (BE)
(72) Inventeur: Balthasart, Dominique, B-1120 Bruxelles (BE); Decap, Philippe, B-1150 Woluwe-St-Pierre (BE)
(74) Mandataire: Jacques, Philippe

(56) Documents cités:
- US-A- 4 021 312
- CHEMICAL ABSTRACTS, vol. 119, no. 11, 13 Septembre 1993 Columbus, Ohio, US; abstract no. 116776, YAN G ET AL 'Preparation, separation and purification of 1,1-difluoroethane' & CN-A-1 069 019 (CHEMICAL INDUSTRY INSTITUTE, ZHEJIANG PROVINCE;PEOP. REP. CHINA) 17 Février 1993

## Description

L'invention concerne un procédé pour séparer du 1,1-difluoroéthane de ses mélanges avec le fluorure d'hydrogène.

Il est connu de préparer le 1,1-difluoroéthane (HFA-152a) par réaction de fluorure d'hydrogène (HF) avec un composé chloré contenant deux atomes de carbone, tel que le chlorure de vinyle, le 1-chloro-1-fluoroéthane ou le 1,1-dichloroéthane.

Le HFA-152a recherché est généralement obtenu en mélange avec des réactifs non transformés et du chlorure d'hydrogène (HCl). La séparation de l'HCl des autres constituants du mélange de produits réactionnels est facilement réalisée par distillation dans une colonne sous pression élevée, de l'ordre de 12 bar ou plus. L'HCl est soutiré en tête de colonne et les autres constituants du mélange des produits réactionnels sont récupérés en pied de colonne. Le HFA-152a doit ensuite être séparé des autres constituants du mélange des produits réactionnels. On a cependant observé qu'il est impossible de séparer complètement le HFA-152a et le HF par distillation dans une colonne opérant sous une pression de l'ordre de 10 bar ou plus, car ces composés forment, dans ces conditions, un azéotrope à température d'ébullition minimale. Il est certes possible d'abattre par de l'eau l'HF contenu dans le mélange azéotropique HF / HFA-152a. Cependant, le procédé de fabrication du HFA-152a nécessitant l'introduction d'HF sous forme anhydre, l'HF abattu à l'eau ne peut pas être recyclé tel quel au réacteur de synthèse, ce qui constitue un inconvénient.

L'invention a pour objet un procédé simple de séparation du HFA-152a de ses mélanges azéotropiques avec l'HF, qui permette à la fois d'obtenir le HFA-152a sous une forme substantiellement pure et de récupérer l'HF sous forme anhydre.

L'invention concerne dès lors un procédé de séparation de 1,1-difluoroéthane d'un mélange de 1,1-difluoroéthane et de fluorure d'hydrogène, en particulier obtenu sous une pression supérieure à 10 bar, dans lequel on soumet le mélange à une distillation dans une colonne fonctionnant à une pression de moins de 10 bar.

De préférence, la colonne de distillation destinée à séparer le 1,1-difluoroéthane de ses mélanges avec le fluorure d'hydrogène fonctionne à une pression inférieure ou égale à 5 bar. Une très bonne séparation est obtenue lorsque la distillation est effectuée à une pression inférieure ou égale à 2 bar.

En effet, l'azéotrope HFA-152a / HF, dont on a décelé l'existence aux pressions supérieures ou égales à 10 bar, disparaît lorsque l'on réduit la pression au-dessous de 10 bar.

La composition des mélanges azéotropiques de HFA-152a et HF à différentes températures et pressions est la suivante :

| Pression (bar) | Température (°C) | HFA-152a (fraction molaire) | HF (fraction molaire) |
|---|---|---|---|
| 23,5 | 80 | 0,97 | 0,03 |
| 15,1 | 60 | 0,99 | 0,01 |
| 8,9 | 40 | pas d'azéotrope | |
| 5,2 | 20 | pas d'azéotrope | |

L'invention met à profil la disparition de l'azéotrope entre le HFA-152a et l'HF à pression réduite, pour séparer le HFA-152a de ses mélanges avec l'HF. Une telle séparation est obtenue en soumettant le mélange à une distillation dans une colonne opérant à une pression de moins de 10 bar (et à une température associée de moins de 43 °C). Dans ces conditions, on récupère en tête de la colonne de distillation du HFA-152a qui renferme une quantité d'HF inférieure à celle de la composition azéotropique existant à pression plus élevée et on récupère en pied de la colonne de distillation du HF sous forme anhydre, substantiellement exempt de HFA-152a. En effectuant la distillation à une pression de moins de 2 bar, on parvient à obtenir en tête de la colonne de distillation du HFA-152a substantiellement débarrassé de tout HF.

Le procédé selon l'invention s'applique particulièrement à la séparation du HFA-152a de ses mélanges avec l'HF non transformé lors de la fabrication du HFA-152a par hydrofluoration de chlorure de vinyle et/ou de 1,1-dichloréthane sous une pression supérieure à 10 bar. Dans un tel procédé, à la sortie du réacteur d'hydrofluoration, le mélange de produits réactionnels contient, outre du HFA-152a et du HF, du chlorure de vinyle et/ou du 1,1-dichloréthane non transformés, du chlorure d'hydrogène (HCl) provenant de l'élimination du chlore du composé de départ, et éventuellement des diluants inertes et divers sous-produits tels que du 1-chloro-1-fluoroéthane (HFA-151a), en faible quantité.

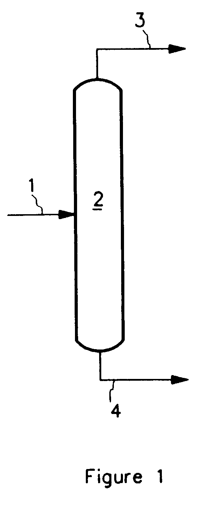

L'invention sera mieux comprise en se référant aux schémas représentés sur les figures 1 à 3 qui représentent trois installations de distillation, appliquées au traitement d'un mélange de produits réactionnels issus d'un réacteur d'hydrofluoration de chlorure de vinyle. Dans ces figures, de mêmes indications de référence désignent des éléments identiques.

Dans une première variante, schématisée à la figure 1, le procédé selon l'invention est appliqué directement à un mélange de produits réactionnels issu d'un réacteur d'hydrofluoration de chlorure de vinyle, un tel mélange comprenant habituellement du HFA-152a produit, de l'HCl co-produit, du HF et du chlorure de vinyle inaltérés, ainsi que du HFA-151a et du 1,1-dichloréthane. Dans cette variante, le mélange des produits réactionnels est introduit par une conduite (1) dans une colonne de distillation (2) fonctionnant à une pression inférieure à 10 bar. L'effluent (3) soutiré en tête de la colonne (2) renferme le HFA-152a et l'HCl. En pied (4) de la colonne (2), on récupère le HF, le chlorure de vinyle, le HFA-151a et le 1,1-dichloréthane qui peuvent être recyclés au réacteur d'hydrofluoration du chlorure de vinyle. Le HFA-152a de l'effluent gazeux (3) peut ensuite être classiquement séparé de l'HCl par distillation.

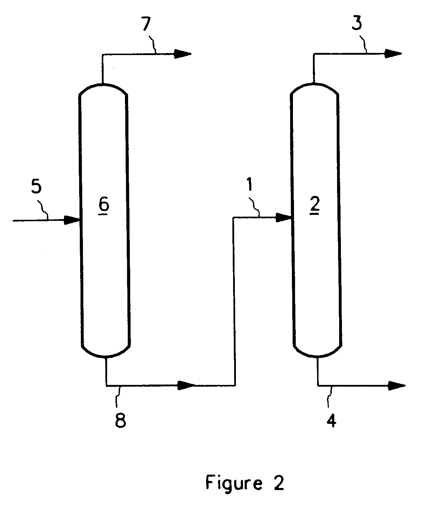

Dans une deuxième variante, schématisée à la figure 2, l'HCl est d'abord séparé du mélange de produits réactionnels. Classiquement, cette variante est mise en oeuvre en introduisant, via une conduite (5), le mélange de produits réactionnels du réacteur d'hydrofluoration dans une colonne de distillation à légers (6), fonctionnant à une pression de plus de 10 bar, en tête de laquelle on soutire, en (7), l'HCl. Les autres composants du mélange sont récupérés en pied (8) de la colonne de distillation à légers (6) et sont introduits par la conduite (1) dans la colonne de distillation (2) fonctionnant, conformément à l'invention, à une pression inférieure à 10 bar. L'effluent (3) soutiré en tête de la colonne (2) contient le HFA-152a. En pied (4) de la colonne (2), on récupère le HF, le chlorure de vinyle, le HFA-151a et le 1,1-dichloréthane qui peuvent être recyclés au réacteur d'hydrofluoration.

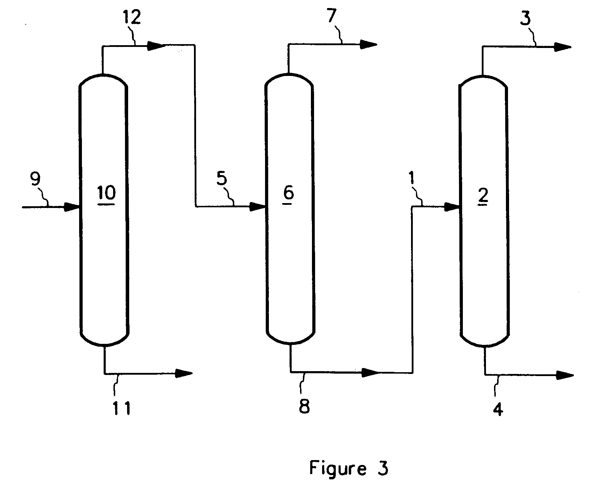

Dans une troisième variante du procédé, schématisée à la figure 3, on introduit d'abord le mélange de produits réactionnels du réacteur d'hydrofluoration, via une conduite (9), dans une colonne de distillation à lourds (10), fonctionnant à une pression de plus de 10 bar. En pied de la colonne (10), on récupère en (11) une partie du HF, le chlorure de vinyle, le HFA-151a et le 1,1-dichloréthane, lesquels peuvent être recyclés au réacteur d'hydrofluoration. En tête de la colonne (10), on soutire, en (12), un effluent contenant le HFA-152a et du HF dans les proportions de la composition azéotropique à la pression de fonctionnement de la colonne (10), ainsi que l'HCl. Cet effluent est introduit via la conduite (5), dans la colonne de distillation à légers (6), fonctionnant également à une pression supérieure à 10 bar. En tête de la colonne (6), on soutire, en (7), l'HCl. Les autres composants du mélange sont récupérés en pied (8) de la colonne (6) et sont introduits par la conduite (1) dans la colonne de distillation (2) fonctionnant, conformément à l'invention, à une pression inférieure à 10 bar. L'effluent (3) soutiré en tête de la colonne (2) contient le HFA-152a. En pied (4) de la colonne (2), on récupère du HF, qui peut être recyclé au réacteur d'hydrofluoration.

La troisième variante du procédé selon l'invention apparait particulièrement avantageuse lorsque le HFA-152a est préparé au départ de chlorure de vinyle et/ou de 1,1-dichloroéthane, dans un réacteur d'hydrofluoration fonctionnant sous pression élevée, de l'ordre de 15 à 30 bar, en phase liquide à l'ébullition. Dans ce cas, la colonne (10) peut avantageusement ne comporter qu'un tronçon de tête, surmontant le réacteur d'hydrofluoration servant de bouilleur.

Les exemples suivants illustrent l'invention de manière non limitative.

### Exemple 1

Dans une colonne de distillation comportant 7 plateaux théoriques, on a introduit un mélange comportant 57 % molaire de HF et 43 % molaire de HFA-152a, sous une pression totale de 8 bar. Le produit obtenu en tête de colonne contenait 1,8 % molaire de HF et 98,2 % molaire de HFA-152a.

### Exemple 2

Dans les mêmes conditions que celles de l'exemple 1, sous une pression totale de 2 bar, le produit obtenu en tête de colonne à reflux total contenait 0,5 % molaire de HF et 99,5 % molaire de HFA-152a.

### Exemple 3

Dans la colonne de distillation de l'exemple 1, on a introduit un mélange comportant 1,2 % molaire de HF et 98,8 % molaire de HFA-152a, sous une pression totale de 2 bar. Le produit obtenu en tête de colonne contenait 0,2 % molaire de HF et 99,8 % molaire de HFA-152a.

## Revendications

1. Procédé de séparation de 1,1-difluoroéthane d'un mélange de 1,1-difluoroéthane et de fluorure d'hydrogène, dans lequel on soumet le mélange à une distillation dans une colonne fonctionnat à une pression de moins de 10 bar.

2. Procédé selon la revendication 1 dans lequel la pression de fonctionnement de la colonne est inférieure ou égale à 5 bar.

3. Procédé selon la revendication 1 dans lequel la pression de fonctionnement de la colonne est inférieure ou égale à 2 bar.

4. Procédé selon une quelconque des revendications 1 à 3, appliqué à la séparation du 1,1-difluoroéthane d'un mélange de 1,1-difluoroéthane et de fluorure d'hydrogène, obtenu par hydrofluoration de chlorure de vinyle ou de 1,1-dichloréthane sous une pression supérieure à 10 bar.

5. Procédé selon la revendication 4, dans lequel du chlorure d'hydrogène généré lors de l'hydrofluoration est éliminé du mélange avant de soumettre le mélange à la distillation.

6. Procédé de séparation selon une quelconque des revendications 1 à 5, dans lequel le mélange à séparer a été obtenu sous une pression supérieure à 10 bar.

7. Procédé de fabrication de 1,1-difluoroéthane par réaction de fluorure d'hydrogène avec du chlorure de vinyle ou du 1,1-dichloroéthane, dans lequel on soumet un mélange de produits réactionnels à une distillation dans une colonne fonctionnant à une pression de moins de 10 bar.

8. Procédé de fabrication de 1,1-difluoroéthane selon la revendication 7 dans lequel
a) on fait réagir du fluorure d'hydrogène avec du chlorure de vinyle ou du 1,1-dichloroéthane pour former un mélange de produits réactionnels comprenant du 1,1-difluoroéthane, du chlorure d'hydrogène, du fluorure d'hydrogène, du chlorure de vinyle, du 1,1-dichloroéthane et du 1-chloro-1-fluoroéthane;
b) on introduit le mélange de produits réactionnels dans une colonne de distillation fonctionnant à une pression inférieure à 10 bar;
c) on soutire en tête de la colonne de distillation un effluent gazeux renfermant le 1,1-difluoroéthane et le chlorure d'hydrogène;
d) on récupère en pied de la colonne de distillation le fluorure d'hydrogène, le chlorure de vinyle, le 1,1-dichloroéthane et le 1-chloro-1-fluoroéthane que l'on recycle éventuellement à l'étape a);
e) on sépare le 1,1-difluoroéthane et le chlorure d'hydrogène de l'effluent gazeux par distillation.

9. Procédé de fabrication de 1,1-difluoroéthane selon la revendication 7 dans lequel
a) on fait réagir du fluorure d'hydrogène avec du chlorure de vinyle ou du 1,1-dichloroéthane pour former un mélange de produits réactionnels comprenant du 1,1-difluoroéthane, du chlorure d'hydrogène, du fluorure d'hydrogène, du chlorure de vinyle, du 1,1-dichloroéthane et du 1-chloro-1-fluoroéthane;
b) on introduit le mélange de produits réactionnels dans une colonne de distillation à légers fonctionnant à une pression de plus de 10 bar;
c) on soutire le chlorure d'hydrogène en tête de la colonne de distillation à légers;
d) on introduit les composants du mélange réactionnel récupérés en pied de la colonne de distillation à légers dans une deuxième colonne de distillation, fonctionnant à une pression inférieure à 10 bar;
e) on soutire en tête de la deuxième colonne de distillation un effluent contenant le 1,1-difluoroéthane;
f) on récupère en pied de la deuxième colonne de distillation le fluorure d'hydrogène, le chlorure de vinyle, le 1,1-dichloroéthane et le 1-chloro-1-fluoroéthane que l'on recycle éventuellement à l'étape a).

10. Procédé de fabrication de 1,1-difluoroéthane selon la revendication 7 dans lequel
a) on fait réagir du fluorure d'hydrogène avec du chlorure de vinyle ou du 1,1-dichloroéthane dans un réacteur d'hydrofluoration pour former un mélange de produits réactionnels comprenant du 1,1-difluoroéthane, du chlorure d'hydrogène, du fluorure d'hydrogène, du chlorure de vinyle, du 1,1-dichloroéthane et du 1-chloro-1-fluoroéthane;
b) on introduit le mélange de produits réactionnels dans une colonne de distillation à lourds fonctionnant à une pression de plus de 10 bar;
c) on récupère en pied de la colonne de distillation à lourds une partie du fluorure d'hydrogène, le chlorure de vinyle, le 1,1-dichloroéthane et le 1-chloro-1-fluoroéthane que l'on recycle éventuellement à l'étape a);
d) on soutire en tête de la colonne de distillation à lourds un effluent contenant le 1,1-difluoroéthane et du fluorure d'hydrogène dans les proportions de la composition azéotropique à la pression de fonctionnement de la colonne de distillation à lourds, ainsi que le chlorure d'hydrogène;
e) on introduit l'effluent soutiré à l'étape d) dans une colonne de distillation à légers fonctionnant à une pression de plus de 10 bar;
f) on soutire le chlorure d'hydrogène en tête de la colonne de distillation à légers;
g) on introduit les autres composants de l'effluent, récupérés en pied de la colonne de distillation à légers dans une troisième colonne de distillation, fonctionnant à une pression inférieure à 10 bar;
h) on soutire en tête de la troisième colonne de distillation un effluent contenant le 1,1-difluoroéthane;
i) on récupère en pied de la troisième colonne de distillation du fluorure d'hydrogène que l'on recycle éventuellement à l'étape a).

11. Procédé selon la revendication 10 dans lequel la colonne de distillation à lourds ne comporte qu'un tronçon de tête, surmontant le réacteur d'hydrofluoration qui lui sert de bouilleur, ledit réacteur fonctionnant sous une pression de 15 à 30 bar.

## Claims

1. Process for separation of 1,1-difluoroethane from a mixture of 1,1-difluoroethane and hydrogen fluoride, in which the mixture is subjected to a distillation in a column operating at a pressure of less than 10 bar.

2. Process according to Claim 1, in which the operating pressure of the column is lower than or equal to 5 bar.

3. Process according to Claim 1, in which the operating pressure of the column is lower than or equal to 2 bar.

4. Process according to any one of Claims 1 to 3, applied to the separation of 1,1-difluoroethane from a mixture of 1,1-difluoroethane and hydrogen fluoride, obtained by hydrofluorination of vinyl chloride or of 1,1-dichloroethane at a pressure higher than 10 bar.

5. Process according to Claim 4, in which hydrogen chloride generated during the hydrofluorination is removed from the mixture before the mixture is subjected to distillation.

6. Process for separation according to any one of Claims 1 to 5, in which the mixture to be separated has been obtained at a pressure higher than 10 bar.

7. Process for the manufacture of 1,1-difluoroethane by reaction of hydrogen fluoride with vinyl chloride or 1,1-dichloroethane, in which a mixture of reaction products is subjected to a distillation in a column operating at a pressure of less than 10 bar.

8. Process for the manufacture of 1,1-difluoroethane according to Claim 7, in which
a) hydrogen fluoride is reacted with vinyl chloride or 1,1-dichloroethane to form a mixture of reaction products comprising 1,1-difluoroethane, hydrogen chloride, hydrogen fluoride, vinyl chloride, 1,1-dichloroethane and 1-chloro-1-fluoroethane;
b) a mixture of reaction products is introduced into a distillation column operating at a pressure of less than 10 bar;
c) a gaseous effluent containing 1,1-difluoroethane and hydrogen chloride is taken off at the top of the distillation column;
d) hydrogen fluoride, vinyl chloride, 1,1-dichloroethane and 1-chloro-1-fluoroethane, which are optionally recycled to step a), are recovered at the bottom of the distillation column;
e) the 1,1-difluoroethane and the hydrogen chloride in the gaseous effluent are separated by distillation.

9. Process for the manufacture of 1,1-difluoroethane according to Claim 7, in which
a) hydrogen fluoride is reacted with vinyl chloride or 1,1-dichloroethane to form a mixture of reaction products comprising 1,1-difluoroethane, hydrogen chloride, hydrogen fluoride, vinyl chloride, 1,1-dichloroethane and 1-chloro-1-fluoroethane;
b) the mixture of reaction products is introduced into a distillation column for light materials operating at a pressure of more than 10 bar;
c) hydrogen chloride is taken off from the top of the distillation column for light materials;
d) the components of the reaction mixture recovered at the bottom of the distillation column for light materials are introduced into a second distillation column operating at a pressure of less than 10 bar;
e) an effluent containing 1,1-difluoroethane is taken off at the top of the second distillation column;
f) hydrogen fluoride, vinyl chloride, 1,1-dichloroethane and 1-chloro-1-fluoroethane, which are optionally recycled to step a), are recovered at the bottom of the second distillation column.

10. Process for the manufacture of 1,1-difluoroethane according to Claim 7, in which
a) hydrogen fluoride is reacted with vinyl chloride or 1,1-dichloroethane in a hydrofluorination reactor in order to form a mixture of reaction products comprising 1,1-difluoroethane, hydrogen chloride, hydrogen fluoride, vinyl chloride, 1,1-dichloroethane and 1-chloro-1-fluoroethane;
b) the mixture of reaction products is introduced into a distillation column for heavy materials operating at a pressure of more than 10 bar;
c) part of the hydrogen fluoride, vinyl chloride, 1,1-dichloroethane and 1-chloro-1-fluoroethane, which are optionally recycled to step a), are recovered at the bottom of the distillation column for heavy materials;
d) an effluent containing 1,1-difluoroethane and hydrogen fluoride in proportions of the azeotropic composition at the operating pressure of the distillation column for heavy materials is taken off at the top of the distillation column for heavy materials, together with hydrogen chloride;
e) the effluent taken off at step d) is introduced into a distillation column for light materials operating at a pressure higher than 10 bar;
f) hydrogen chloride is taken off at the top of the distillation column for light materials;
g) the other components of the effluent recovered at the bottom of the distillation column for light materials are introduced into a third distillation column operating at a pressure of less than 10 bar;
h) an effluent containing 1,1-difluoroethane is taken off at the top of the third distillation column;
i) hydrogen fluoride, which is optionally recycled to step a), is recovered at the bottom of the third distillation column.

11. Process according to Claim 10, in which the distillation column for heavy materials comprises only a top section mounted above the hydrofluorination reactor used as boiler for it, the said reactor operating at a pressure of 15 to 30 bar.

## Patentansprüche

1. Verfahren zur Abtrennung von 1,1-Difluorethan aus einem Gemisch von 1,1-Difluorethan und Fluorwasserstoff, bei dem man das Gemisch einer Destillation in einer Kolonne, die bei einem Druck Von weniger als 10 bar arbeitet, unterzieht.

2. Verfahren gemäß Anspruch 1, bei dem der Betriebsdruck der Kolonne niedriger oder gleich 5 bar ist.

3. Verfahren gemäß Anspruch 1, bei dem der Betriebsdruck der Kolonne niedriger oder gleich 2 bar ist.

4. Verfahren gemäß einem der Ansprüche 1 bis 3, angewendet auf die Abtrennung des 1,1-Difluorethans aus einem Gemisch von 1,1-Difluorethan und Fluorwasserstoff, das durch Hydrofluorierung von Vinylchlorid oder von 1,1-Dichlorethan unter einem Druck, der höher als 10 bar ist, erhalten wurde.

5. Verfahren gemäß Anspruch 4, bei dem Chlorwasserstoff, der bei der Hydrofluorierung erzeugt wurde, aus dem Gemisch entfernt wird, bevor das Gemisch destilliert wird.

6. Verfahren zur Abtrennung gemäß einem der Ansprüche 1 bis 5, bei dem das zu trennende Gemisch unter einem Druck, der höher als 10 bar ist, erhalten wurde.

7. Verfahren zur Herstellung von 1,1-Difluorethan durch Reaktion von Fluorwasserstoff mit Vinylchlorid oder 1,1-Dichlorethan, bei dem man ein Gemisch von Reaktionsprodukten einer Destillation in einer Kolonne, die bei einem Druck von weniger als 10 bar arbeitet, unterzieht.

8. Verfahren zur Herstellung von 1,1-Difluorethan gemäß Anspruch 7, bei dem
a) man Fluorwasserstoff mit Vinylchlorid oder 1,1-Dichlorethan umsetzt, um ein Gemisch von Reaktionsprodukten zu bilden, das 1,1-Difluorethan, Chlorwasserstoff, Fluorwasserstoff, Vinylchlorid, 1,1-Dichlorethan und 1-Chlor-1-fluorethan umfaßt;
b) man das Gemisch von Reaktionsprodukten in eine Destillationskolonne einführt die bei einem Druck, der niedriger als 10 bar ist, arbeitet;
c) man am Kopf der Destillationskolonne einen gasförmigen Ausstoß entnimmt, der das 1,1-Difluorethan und den Chlorwasserstoff enthält;
d) man am Fuß der Destillationskolonne den Fluorwasserstoff, das Vinylchlorid, das 1,1-Dichlorethan und das 1-Chlor-1-fluorethan gewinnt, die man gegebenenfalls in den Schritt a) zurückführt;
e) man das 1,1-Difluorethan und den Fluorwasserstoff aus dem gasförmigen Ausstoß durch Destillation abtrennt.

9. Verfahren zur Herstellung von 1,1-Difluorethan gemäß Anspruch 7, bei dem
a) man Fluorwasserstoff mit Vinylchlorid oder 1,1-Dichlorethan umsetzt, um ein Gemisch von Reaktionsprodukten zu bilden, das 1,1-Difluorethan, Chlorwasserstoff, Fluorwasserstoff, Vinylchlorid, 1,1-Dichlorethan und 1-Chlor-1-fluorethan umfaßt;
b) man das Gemisch von Reaktionsprodukten in eine Leichtsiederdestillationskolonne einführt, die bei einem Druck von mehr als 10 bar arbeitet;
c) man am Kopf der Leichtsiederdestillationskolonne den Chlorwasserstoff entnimmt;
d) man die Bestandteile des Reaktionsgemischs, die am Fuß der Leichtsiederdestillationskolonne gewonnen wurden, in eine zweite Destillationskolonne einführt, die bei einem Druck, der niedriger als 10 bar ist, arbeitet;
e) man am Kopf der zweiten Destillationskolonne einen Ausstoß entnimmt, der das 1,1-Difluorethan enthält;
f) man am Fuß der zweiten Destillationskolonne den Fluorwasserstoff, das Vinylchlorid, das 1,1-Dichlorethan und das 1-Chlor-1-fluorethan gewinnt, die man gegebenenfalls in den Schritt a) zurückführt.

10. Verfahren zur Herstellung von 1,1-Difluorethan gemäß Anspruch 7, bei dem
a) man Fluorwasserstoff mit Vinylchlorid oder 1,1-Dichlorethan in einem Hydrofluorierungsreaktor umsetzt, um ein Gemisch von Reaktionsprodukten zu bilden, das 1,1-Difluorethan, Chlorwasserstoff, Fluorwasserstoff, Vinylchlorid, 1,1-Dichlorethan und 1-Chlor-1-fluorethan umfaßt;
b) man das Gemisch von Reaktionsprodukten in eine Schwersiederdestillationskolonne einführt, die bei einem Druck von mehr als 10 bar arbeitet;
c) man am Fuß der Schwersiederdestillationskolonne einen Teil des Fluorwasserstoffs, das Vinylchlorid, das 1,1-Dichlorethan und das 1-Chlor-1-fluorethan gewinnt, die man gegebenenfalls in den Schritt a) zurückführt;
d) man am Kopf der Schwersiederdestillationskolonne einen Ausstoß entnimmt, der das 1,1-Difluorethan und Fluorwasserstoff in den Verhältnissen des azeotropen Gemischs beim Betriebsdruck der Schwersiederdestillationskolonne sowie den Chlorwasserstoff enthält;
e) man den im Schritt d) entnommenen Ausstoß in eine Leichtsiederdestillationskolonne einführt, die bei einem Druck von mehr als 10 bar arbeitet;
f) man den Chlorwasserstoff am Kopf der Leichtsiederdestillationskolonne entnimmt;
g) man die anderen Bestandteile des Ausstoßes, die am Fuß der Leichtsiederdestillationskolonne gewonnen wurden, in eine dritte Destillationskolonne einführt, die bei einem Druck, der niedriger als 10 bar ist, arbeitet;
h) man am Kopf der dritten Destillationskolonne einen Ausstoß entnimmt, der das 1,1-Difluorethan enthält;
i) man am Fuß der dritten Destillationskolonne Fluorwasserstoff gewinnt, den man gegebenenfalls in den Schritt a) zurückführt.

11. Verfahren gemäß Anspruch 10, bei dem die Schwersiederdestillationskolonne nur ein Kopfstück umfaßt, das über dem Hydrofluorierungsreaktor angeordnet ist, der als Siedekessel dient, wobei besagter Reaktor unter einem Druck von 15 bis 30 bar arbeitet.
